(19) Europäisches Patentamt
European Patent Office
Office européen des brevets

(11) **EP 4 435 094 A1**

(12) **EUROPEAN PATENT APPLICATION**
published in accordance with Art. 153(4) EPC

(43) Date of publication:
**25.09.2024 Bulletin 2024/39**

(21) Application number: **22895627.2**

(22) Date of filing: **16.11.2022**

(51) International Patent Classification (IPC):
**C12N 5/0775** (2010.01)

(52) Cooperative Patent Classification (CPC):
**C12N 5/06**

(86) International application number:
**PCT/JP2022/042503**

(87) International publication number:
**WO 2023/090346 (25.05.2023 Gazette 2023/21)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **18.11.2021 JP 2021188216**

(71) Applicant: **National University Corporation Tokyo
Medical
and Dental University
Tokyo 113-8510 (JP)**

(72) Inventors:
• **IWATA Takanori
Tokyo 113-8510 (JP)**
• **ONIZUKA Satoru
Tokyo 113-8510 (JP)**

(74) Representative: **Mewburn Ellis LLP
Aurora Building
Counterslip
Bristol BS1 6BX (GB)**

(54) **CELL POPULATION INCLUDING MESENCHYMAL STEM CELLS, CELL SHEET, AND CELL SHEET PRODUCTION METHOD**

(57)    The present invention is directed to obtaining a cell population comprising mesenchymal stem cells that are excellent in proliferative capability and osteoblastic differentiation capability and are useful for the regeneration of periodontal tissues. The present invention is also directed to providing a cell sheet and a method for producing a cell sheet, comprising, as a material, a cell population comprising mesenchymal stem cells that are excellent in proliferative capability and osteoblastic differentiation capability and are useful for the regeneration of periodontal tissues. A cell population having alkaline phosphatase activity before calcification induction of 1 U or more is selected and obtained from a cell population comprising mesenchymal stem cells derived from a dental or periodontal tissue. A cell sheet is obtained by the following steps (a) to (c): (a) obtaining a cell population comprising mesenchymal stem cells and having alkaline phosphatase activity; (b) culturing the cell population in a calcification induction medium, and proliferating the cell population on a plastic substrate; and (c) recovering and washing a sheet-shaped cell population formed on the plastic substrate.

Fig. 1

**Description**

Technical Field

The present invention relates to a cell population comprising mesenchymal stem cells, a cell sheet, and a method for producing a cell sheet.

Background Art

Mesenchymal stem cells (MSCs) included in stromal cells typically refer to stem cells having the ability to differentiate into mesoderm-derived tissues such as bone, cartilage, blood vessel, and cardiomyocytes. The International Society for Cellular Therapy has proposed, as criteria for defining human MSCs, the following minimum requirements: (1) having adherence to plastics under standard culture conditions; (2) being positive to cell surface markers CD73, CD90, and CD105 and negative to CD79α, CD34, CD45, and HLA-DR; and (3) having differentiation capability to osteoblastic, chondrocytic, or adipocytic (Non Patent Literature 1).
MSCs are known to have an anti-inflammatory effect, a growth factor-inducing effect, an angiogenesis-promoting effect, and the like and to play an important role in tissue repair. The application of MSCs to regenerative medicine has been underway in recent years, and practical use of new treatment methods that promote the regeneration or functional improvement of tissues or organs has progressed rapidly. For example, Patent Literature 1 discloses a sheet of periodontal ligament-derived cells comprising MSCs, the sheet being usable in the regeneration of a periodontal tissue damaged by periodontal disease or the like.

Citation List

Patent Literature

Patent Literature 1: International Publication No. WO 2005/103233

Non Patent Literature

Non Patent Literature 1: M. Dominici, MD et al., Cytotherapy: 8, 315-317 (2006)

Summary of Invention

Technical Problem

A plurality of useful markers for evaluating MSCs have been proposed. For example, as described above, Non Patent Literature 1 discloses markers essential for MSCs, such as CD105, CD73, and CD90. However, more useful markers are demanded because cells expressing these markers do not always exhibit favorable proliferation.
MSCs differ largely in its proliferative capability and differentiation capability depending on a source tissue. Therefore, a useful marker for MSCs evaluation may also differ depending on the source tissue or purpose of use of MSCs. Patent Literature 1 discloses a method for producing a cell sheet of periodontal ligament-derived cells, but does not disclose a marker or the like for selecting cells serving as a material.
An object of the present invention is to obtain a cell population comprising mesenchymal stem cells that are excellent in proliferative capability and osteoblastic differentiation capability and are useful for the regeneration of periodontal tissues. Another object of the present invention is to provide a cell sheet and a method for producing a cell sheet, comprising, as a material, a cell population comprising mesenchymal stem cells that are excellent in proliferative capability and osteoblastic differentiation capability and are useful for the regeneration of periodontal tissues.

Summary of the Invention

The present inventors have searched for and found a marker capable of identifying MSCs excellent in proliferative capability and osteoblastic differentiation capability before differentiation induction for MSCs to be applied to the regeneration of periodontal tissues. The present inventors have further found that MSCs having high periodontal tissue regeneration capability can be obtained by using the marker as an index for selection; and a cell sheet useful for periodontal tissue regeneration can be obtained by using these MSCs as a material. Based on these findings, the present invention has been completed.
Specifically, the present specification provides the following aspects of the invention.

(1) A cell population comprising mesenchymal stem cells derived from a dental or periodontal tissue, wherein alkaline phosphatase activity before calcification induction is 1 U or more.

(2) The cell population according to (1), wherein alkaline phosphatase activity after calcification induction is 5 U or more.

(3) The cell population according to (1) or (2), wherein the mesenchymal stem cells are mesenchymal stem cells derived from periodontal ligament.

(4) A cell sheet comprising a cell population comprising mesenchymal stem cells derived from a dental or periodontal tissue, wherein alkaline phosphatase activity after calcification induction of the cell population is 5 U or more.

(5) The cell sheet according to (4), wherein alkaline phosphatase activity before calcification induction of the cell population is 1 U or more.

(6) A method for producing a cell sheet, comprising the following steps (a) to (c):

(a) obtaining a cell population comprising mesenchymal stem cells derived from a dental or periodontal tissue and having alkaline phosphatase activity;

(b) culturing the cell population in a calcification induction medium, and proliferating the cell population on a plastic substrate; and

(c) recovering and washing a sheet-shaped cell population formed on the plastic substrate.

(7) The method according to (6), wherein alkaline phosphatase activity of the cell population in the step (a) is 1 U or more.

(8) The method according to (6) or (7), wherein alkaline phosphatase activity after calcification induction of the cell population is 5 U or more.

(9) A method for preserving and/or transporting a cell sheet comprising a cell population comprising mesenchymal stem cells derived from a dental or periodontal tissue, wherein the cell population has high alkaline phosphatase activity, and the cell sheet is maintained in alpha modified Eagle's minimum essential medium at ordinary temperature.

(10) The method according to (9), wherein the cell sheet is capable of being maintained in a usable state for 7 days or longer in the alpha modified Eagle's minimum essential medium at ordinary temperature.

(11) The method according to (9) or (10), wherein the cell sheet is a cell sheet according to (4) or (5) or a cell sheet produced by a method according to any of (6) to (8).

The present specification encompasses the contents disclosed in Japanese Patent Application No. 2021-188216 on which the priority of the present application is based.

Advantageous Effects of Invention

The present invention is capable of obtaining a cell population comprising mesenchymal stem cells that are excellent in proliferative capability and osteoblastic differentiation capability and are useful for the regeneration of periodontal tissues. The present invention is also capable of providing a cell sheet and a method for producing a cell sheet, comprising, as a material, a cell population comprising mesenchymal stem cells that are excellent in proliferative capability and osteoblastic differentiation capability and are useful for the regeneration of periodontal tissues.

Brief Description of Drawings

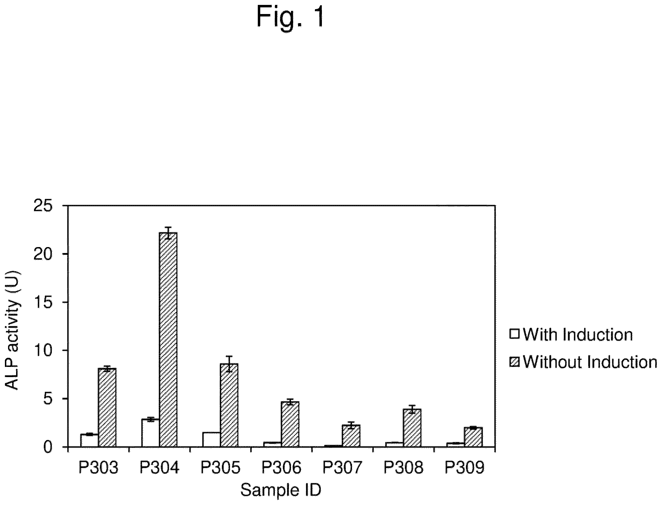

[Figure 1] Figure 1 is a graph showing results of measuring alkaline phosphatase activity of each cell population comprising MSCs before calcification induction and after calcification induction.

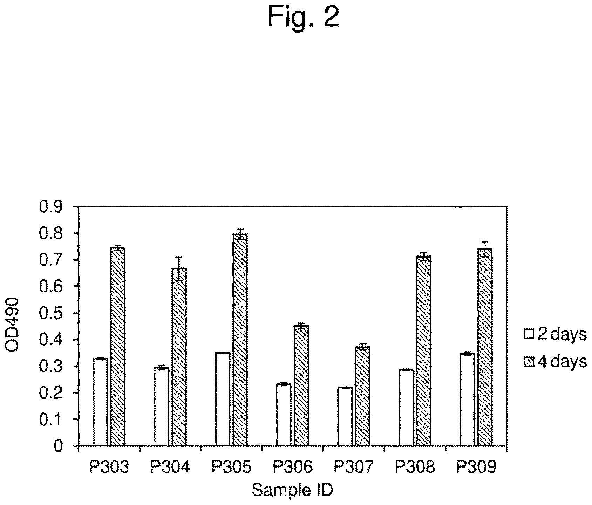

[Figure 2] Figure 2 is a graph showing results of measuring cellular proliferative capability of each cell population comprising MSCs before calcification induction.

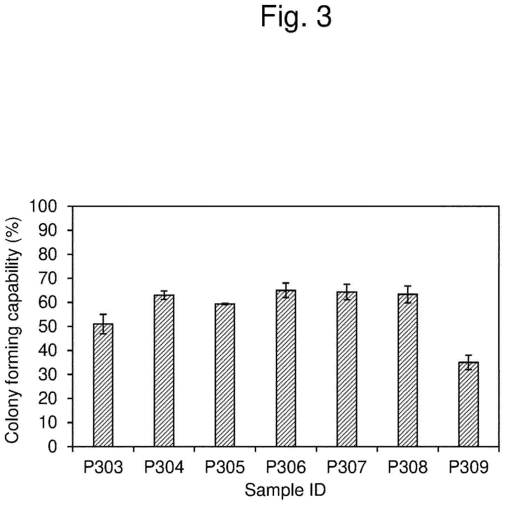

[Figure 3] Figure 3 is a graph showing results of measuring colony forming capability of each cell population comprising MSCs before calcification induction.

Description of Embodiments

[1] Cell population comprising mesenchymal stem cell

A cell population comprising mesenchymal stem cells (MSCs) according to the present invention (hereinafter, also referred to as the "cell population of the present invention") is a cell population comprising MSCs derived from a dental or periodontal tissue, wherein alkaline phosphatase activity (hereinafter, the alkaline phosphatase is also referred to as the "ALP", and the alkaline phosphatase activity is also referred to as the "ALP activity") before calcification induction is 1 U or more. The cell population of the present invention has the advantages of being excellent in proliferative capability and osteoblastic differentiation capability and being useful for the regeneration of periodontal tissues. The cell population of the present invention also has the advantage of being able to be used in the production of a cell sheet, particularly, a cell sheet useful for the regeneration of periodontal tissues.

In the present invention, the "mesenchymal stem cells (MSCs)" refer to cells that satisfy the following definitions among somatic cells (tissue cells) which can be collected from various tissues and organs:

(1) having adherence to plastics under standard culture conditions;
(2) being positive in cell surface markers CD73, CD90, and CD105 and negative in CD79$\alpha$, CD34, CD45, and HLA-DR; and
(3) having differentiation capability to osteoblastic, chondrocytic, or adipocytic.

In the present invention, the "cell population comprising MSCs" refers to a cell population mainly comprising MSCs. In this context, the state "mainly comprising MSCs" refers to a state in which 90% or more, particularly, 95% or more, of cells satisfy the condition of "being positive in CD73, CD90, and CD105 and negative in CD79$\alpha$, CD34, CD45, and HLA-DR" in flow cytometry analysis. As for the origin of the cell population comprising MSCs, the cell population comprises MSCs derived from a dental or periodontal tissue. Examples of the MSCs derived from a dental or periodontal tissue include MSCs derived from dental pulp, periodontal ligament, dental follicle, dental papilla, or primary teeth. Particularly, MSCs derived from periodontal ligament is preferred. Hereinafter, embodiments using MSCs derived from periodontal ligament are described as examples and however, do not intend to limit the scope of the present invention.

In the present invention, the "ALP activity" of the cell population before calcification induction refers to enzymatic activity that is measured by the following approach: the cell population before calcification induction is seeded at $1 \times 10^4$ cells/well (100 $\mu$L) to a 96-well microplate. The cell population is cultured in a basal medium (alpha modified Eagle's minimum essential medium (oc-MEM) supplemented with 10% FBS) at 37°C in a 5% $CO_2$ environment for 2 days. After medium replacement, the cell population is further cultured for 5 days. The medium is removed, and the cells are washed. Then, a substrate (e.g., p-nitrophenyl phosphate (pNPP)) buffer solution is added thereto and reacted under a condition of 37°C. If necessary, a stop solution is added thereto, followed by the measurement of absorbance. The ALP activity of the cell population is calculated using a calibration curve prepared from measurement values of p-nitrophenol (pNP) solutions having a known concentration. Specifically, the ALP activity (U) described herein refers to the amount of activity in the cell population thus cultured for 7 days under predetermined conditions from an initial cell number of $1 \times 10^4$ cells.

A specific method for measuring the ALP activity (U) is not particularly limited, but the ALP activity can be calculated by, for example, the following method: pNP standard solutions having a plurality of concentrations are dispensed to wells of a microwell plate, and absorbances at 405 nm are measured to determine the following calibration curve.

$$Y = aX + b$$

wherein X represents a pNP concentration (mmol/L), and Y represents absorbance at 405 nm.

The ALP activity of a sample is calculated from measured absorbance at 405 nm through the reaction of 100 $\mu$L of the sample with a pNPP solution for 5 minutes. The absorbance is substituted into Y of the calibration curve to calculate concentration X (mmol/L) of pNP formed by ALP of the sample. Subsequently, ALP activity per $\mu$L (unit/$\mu$L) is calculated. In the present specification, 1 unit of the ALP activity refers to the activity of the enzyme that forms 1 nmol of pNP at pH 9.8 at 37°C for 1 minute.

$$\text{ALP activity (unit/}\mu\text{L)} = \text{X (mmol/L)} / 5 \text{ (min)}$$

The total amount of activity (U) of 100 $\mu$L of the sample is calculated according to the following expression.

$$\text{ALP activity (U)} = \text{ALP activity (unit/}\mu\text{L)} \times 100$$

In the present invention, the ALP activity of the cell population before calcification induction is 1 U or more, preferably 1.2 U or more, more preferably 1.4 U or more, further preferably 2 U or more.

In the present invention, the ALP activity of the cell population after calcification induction is preferably 5 U or more, more preferably 7 U or more, further preferably 8.5 U or more, still further preferably 15 U or more. The "ALP activity after calcification induction" refers to the ALP activity of the cell population obtained by culturing the cell population of the present invention in a calcification induction medium.

Any medium commercially available as a general calcification induction medium may be used as the "calcification induction medium". For example, a medium obtained by adding L-ascorbic acid phosphate magnesium salt n-hydrate, dexamethasone, β-glycerophosphoric acid, and the like to a basal medium (e.g., α-MEM supplemented with 10% FBS) can be used.

Conditions for culturing the cell population comprising MSCs using the calcification induction medium are not particularly limited as long as the conditions enable MSCs to be sufficiently induced and proliferated. The conditions can be as follows, for example: a cell suspension selected with an index of the ALP activity before calcification induction is seeded at $1 \times 10^4$ cells/well (100 μL) to a 96-well microplate. The cells are cultured in a basal medium (alpha modified Eagle's minimum essential medium (oc-MEM) supplemented with 10% FBS) at 37°C in a 5% $CO_2$ environment for 2 days. Subsequently, the medium is replaced with a calcification induction medium (e.g., α-MEM containing L-ascorbic acid phosphate magnesium salt n-hydrate, dexamethasone, β-glycerophosphoric acid, and the like), and the cells are further cultured at 37°C in a 5% $CO_2$ environment for 5 days. The ALP activity of the resulting cell population after calcification induction is measured by use of the same approach as that for the cell population before calcification induction.

The ALP activity before calcification induction of MSCs derived from periodontal ligament is usually lower than its ALP activity after calcification induction and differs relatively small among individuals. However, among others, a cell population comprising MSCs that exhibits higher ALP activity before calcification induction has been confirmed to have high proliferative capability. Also, such cells have exhibited marked increase in ALP activity after calcification induction and have been confirmed to have high osteoblastic differentiation capability. Thus, a cell population comprising MSCs having high proliferative capability and osteoblastic differentiation capability can be selected and obtained by using the ALP activity as an index.

The obtained cell population comprising MSCs exhibit high cellular proliferative capability and colony forming capability. In this context, the "cellular proliferative capability" can be measured by, for example, the following approach: $1 \times 10^3$ cells are seeded to each well of a 96-well plate and cultured in a basal medium at 37°C in a 5% $CO_2$ environment for 2 days and 4 days. The medium is replaced with 100 μL of a fresh basal medium, followed by measurement using CellTiter 96 AQueous One Solution Cell Proliferation Assay (Promega Corp.).

The "colony forming capability" can be measured by, for example, the following approach: 100 cells are seeded to a 60 cm$^2$ Petri dish and cultured in a basal medium at 37°C in a 5% $CO_2$ for 14 days. During this period, medium replacement is carried out twice a week. Then, the cells are stained with crystal violet in accordance with a routine method. Among the seeded 100 cells, the number of cells that have formed a colony, i.e., a colony number, is counted, and its ratio (%) is used as an index for the colony forming capability.

The cellular proliferative capability and the colony forming capability may be measured by a method described in, for example, Iwata et al., J. Clin. Periodontol., Vol. 37, pp. 1088-1099 (2010).

[2] Cell sheet

The cell sheet of the present invention is a cell sheet comprising a cell population comprising MSCs, wherein ALP activity after calcification induction of the cell population is 5 U or more. In this context, the cell population described in the section "[1] Cell population comprising mesenchymal stem cell" can be used as the "cell population comprising MSCs". The cell sheet of the present invention has high ALP activity and has high engraftment capability and regeneration capability for periodontal tissues, and thereby has the advantage that the cell sheet is useful, particularly, for the regeneration of periodontal tissues.

In the present invention, the "cell sheet" refers to a material obtained by detaching cells attached in a confluent state onto a plastic substrate, with their sheet shape maintained, or by layering the cells in a sheet shape. Particularly, the cells are proliferated on a temperature-responsive cell cultureware (e.g., UpCell(R) (manufactured by CellSeed Inc.)) so that the cells can be easily detached in a sheet shape by mere temperature change without being subjected to enzymatic treatment or the like. Such cells maintain the original functions of the cells because of the absence of enzymatic treatment. The cells also retain a cell adhesion protein and therefore have the advantage of being more rapidly engrafted when applied to a living body.

The "plastic" described herein refers to a synthetic resin having plasticity. The plastic is not particularly limited as long as the plastic is a synthetic resin material that can be formed into a flat plate shape. For example, any of polyethylene, polystyrene, polypropylene, polycarbonate, and poly(meth)acrylamide can be used. Specific examples thereof include materials obtained by the homopolymerization or copolymerization of monomers such as (meth)acrylamide compounds and N- (or N,N-di)alkyl-substituted (meth)acrylamide derivatives described in JP Patent Publication (Kokai) No. 02-211865 A (1990). Particularly, a temperature-responsive cell cultureware (e.g., UpCell(R)) can be preferably used which can detach adhering cells by temperature change.

The ALP activity after calcification induction of the cell population comprising MSCs, which constitutes the cell sheet of the present invention, is 5 U or more, preferably 7 U or more, more preferably 8.5 U or more, further preferably 15 U or more. The ALP activity before calcification induction of the cell population is preferably 1 U or more, more preferably 1.2 U or more, further preferably 1.4 U or more. In this context, the ALP activity values are defined as described in the section "[1] Cell population comprising mesenchymal stem cell", and induction of calcification and selection/obtainment can also be performed by the methods described in the section "[1] Cell population comprising mesenchymal stem cell".

The cell sheet of the present invention can be produced by a method described in the section "[3] Method for producing cell sheet" mentioned later. More specifically, the cell sheet of the present invention is a cell sheet produced by a method described in the section "[3] Method for producing cell sheet", and detailed conditions such as the properties, material, and production method of the cell sheet are as described in the sections "[1] Cell population comprising mesenchymal stem cell" and "[3] Method for producing cell sheet" unless otherwise specified.

[3] Method for producing cell sheet

The method for producing a cell sheet according to the present invention (hereinafter, also simply referred to as the "method of the present invention") comprises the following steps (a) to (c):

(a) obtaining a cell population comprising MSCs derived from a dental or periodontal tissue and having ALP activity;
(b) culturing the cell population in a calcification induction medium, and proliferating the cell population on a plastic substrate; and
(c) recovering and washing a formed sheet-shaped cell population.

The method of the present invention has the features described above and is capable of producing a cell sheet useful, particularly, for the regeneration of periodontal tissues, using MSCs excellent in proliferative capability and osteoblastic differentiation capability as a material.

[3-1] Step of obtaining cell population comprising MSCs (step (a))

The method of the present invention comprises the step of obtaining a cell population comprising MSCs and having ALP activity (hereinafter, also referred to as the "step (a)").

The "cell population comprising MSCs and having ALP activity" obtained in this step is the cell population comprising MSCs derived from a dental or periodontal tissue (preferably MSCs derived from periodontal ligament) as described in the section "[1] Cell population comprising mesenchymal stem cell", the cell population having been selected and obtained by using the following index: ALP activity before calcification induction measured by the method described in the section "[1] Cell population comprising mesenchymal stem cell" exhibits a certain value or more.

In the step (a), the ALP activity as an index for selecting and obtaining the cell population, i.e., the ALP activity of the cell population before calcification induction, is preferably 1 U or more, more preferably 1.2 U or more, further preferably 1.4 U or more.

[3-2] Step of culturing cell population comprising MSCs in calcification induction medium (step (b))

The method of the present invention comprises the step of culturing the cell population comprising MSCs obtained in the step (a) in a calcification induction medium, and proliferating the cell population on a plastic substrate (hereinafter, also referred to as the "step (b)").

In the step (b), the culture of the cell population is performed in a container having a plastic substrate. A container in which the inside of its bottom and/or wall is formed from a flat plastic is used, and the inner face of the bottom and/or the wall may be a plastic substrate. Alternatively, aside from a material constituting the container, a plastic material formed in a flat plate shape may be disposed in the container and allowed to function as a plastic substrate. The plastic substrate is preferably formed by the inner face of the bottom and/or the wall of the container, more preferably the inner face of the bottom of the container.

Particularly, a temperature-responsive cell cultureware (e.g., UpCell(R)) can be preferably used as a material of the plastic substrate for use in the step (b). In the step (b), examples of the container for use in culture include microwell plates, Petri dishes, and tissue culture flasks. Particularly, a microwell plate, a Petri dish, or the like having a temperature-responsive cell cultureware in its bottom or wall can be preferably used. Hereinafter, embodiments using a Petri dish having a temperature-responsive cell cultureware are described as examples, however, do not intend to limit the scope of the present invention.

Any medium commercially available as a general calcification induction medium may be used as the calcification induction medium for use in the step (b). For example, a medium obtained by adding L-ascorbic acid phosphate magnesium salt n-hydrate, dexamethasone, and β-glycerophosphoric acid to a basal medium (e.g., α-MEM supplemented with 10% FBS) can be used.

Culture conditions in culturing the cell population comprising MSCs using the calcification induction medium are not particularly limited as long as the conditions enable MSCs to be sufficiently induced and proliferated. The conditions can be as follows, for example.

(1) Seeding

A cell suspension selected with an index of the ALP activity before calcification induction is thawed, precultured, if necessary, and then seeded in the concentration of $1 \times 10^4$ to $1 \times 10^6$ cells/cm$^2$, preferably $1 \times 10^4$ to $1 \times 10^5$ cells/cm$^2$, to a calcification induction medium (e.g., α-MEM compriding L-ascorbic acid phosphate magnesium salt n-hydrate, dexamethasone, and β-glycerophosphoric acid) in a Petri dish.

(2) Culture/calcification induction

Culture can be carried out in the calcification induction medium, for example, under conditions of 37°C and 5% CO$_2$. The culture is preferably performed for approximately 7 to 10 days while medium replacement is performed every 3 to 4 days. By the culture, the cell population comprising MSCs is usually proliferated in a state adhering onto the plastic substrate in the culture system, because of its properties, and forms a sheet shape.

The cell population comprising MSCs after calcification induction in the step (b) preferably has higher ALP activity than that before calcification induction. The ALP activity after calcification induction is particularly preferably twice or more. or more preferably three or more times the ALP activity before the induction. The ALP activity of the cell population comprising MSCs after calcification induction, when measured by the method described above, is preferably 5 U or more, more preferably 7 U or more, further preferably 8.5 U or more, still further preferably 15 U or more.

[3-3] Step of recovering and washing sheet-shaped cell population

The method of the present invention comprises the step of recovering and washing a sheet-shaped cell population formed on the plastic substrate in the step (b) (hereinafter, also referred to as the "step (c)").

The cell population after calcification induction in the step (b) is formed into a sheet shape in a state adhering onto the plastic substrate. The sheet-shaped cell population adhering thereonto may be detached by any method known in the art such as trypsin treatment or scraper treatment as long as the conditions enable the sheet shape to be maintained. In the case of using a temperature-responsive cell cultureware as the plastic substrate, the cell population can be detached, without degrading cell adhesion factors, by the application of temperature change to the medium.

The sheet-shaped cell population thus detached is washed with a washing solution. Physiological saline, PBS, a serum-free basal medium, or the like can be used as the washing solution for use in the step (c). The washed sheet is placed in a preservation container containing a preservation solution. Another sheet of a cell population may be washed and further layered on the sheet previously placed. The sheets are preferably layered into 2 to 5 layers, for example, 3 layers. A housing container that can be used is a housing container as described in, for example, JP Patent Publication (Kokai) No. 2018-121568 A (2018). The preservation solution to be contained in the preservation container is preferably α-MEM.

[4] Method for preserving and/or transporting cell sheet

The present invention provides a method for preserving and/or transporting a cell sheet comprising a cell population comprising MSCs derived from a dental or periodontal tissue (hereinafter, also referred to as the "preservation/transport method"). The cell population constituting the cell sheet to be preserved and/or transported by the preservation/transport method of the present invention has high ALP activity. In the preservation/transport method of the present invention, the cell sheet is maintained in α-MEM and preserved and/or transported at ordinary temperature.

The cell sheet may be housed in a dedicated housing container and preserved and transported. Heretofore, the cell sheet has been preserved and transported under a low-temperature condition on the order of 2 to 10°C, particularly, 2 to 5°C. This requires cost and labor, such as the provision of refrigeration equipment. Preservation and transport at ordinary temperature are desirable for more simple handling of the cell sheet. The "ordinary temperature" described herein refers to a general temperature without warming or cooling in a usual living environment. The ordinary temperature is preferably 15 to 25°C under preservation and transport conditions for the cell sheet.

The present inventors have found that the cell sheet to be preserved and/or transported is maintained in a usable state at ordinary temperature by immersing the cell sheet in α-MEM. In the preservation/transport method of the present invention, the cell sheet is capable of being maintained in a usable state even if preserved in α-MEM at ordinary temperature for 7 days or longer, preferably 10 days or longer. However, the actual preservation period is preferably 7 days or shorter, particularly, 5 days or shorter.

In the present invention, the cell sheet "in a usable state" specifically refers to a cell sheet having a cell number, a cell survival rate, ALP activity, and ALP purity all equal to or higher than preset values of standard. Their respective preset values of standard are as follows.

Cell number: $1 \times 10^6$ cells/product (upper face: circle having a diameter of 21 mm)
Cell survival rate: 80%
ALP activity: 10 U or more
ALP-positive cell ratio: 50%

The "ALP activity" of the cell sheet refers to ALP activity measured for a cell sheet piece recovered from a cell sheet product using a biopsy punch ($\phi$8.0 mm).

The "cell number" and the "cell survival rate" of the cell sheet can be measured by, for example, the following approach: the remaining portion of the cell sheet recovered using a biopsy punch is treated with collagenase and subsequently treated with trypsin to prepare a cell suspension. The cells are washed and then stained with trypan blue, and the cell number and survival rate are confirmed under a microscope.

The "ALP-positive cell ratio" can be measured using FACS after reaction of a cell suspension recovered from the cell sheet with an anti-ALP antibody.

The "cell number", the "cell survival rate", and the "ALP-positive cell ratio" of the cell sheet can be measured by methods described in, for example, Washio, et al., Int. J. Oral Maxillofac. Implants, vol. 29, No. 1, e117-121 (2014).

In the preservation/transport method of the present invention, the cell sheet to be preserved and/or transported is preferably the cell sheet described in the section "[2] Cell sheet" or the cell sheet produced by the method described in the section "[3] Method for producing cell sheet". The properties, material, production method, and the like of the cell sheet to be preserved are as described in the sections "[1] Cell population comprising mesenchymal stem cell", "[2] Cell sheet", and "[3] Method for producing cell sheet" unless otherwise specified.

Examples

Hereinafter, the present invention is described in more detail with reference to Examples. However, the present invention does not intend to be limited by the scope of Examples.

[Example 1] Collection, isolation, and culture of periodontal ligament tissue cell

Samples were collected from seven healthy donors (P303 to P309) who gave informed consent under approval of an ethical committee. One third molar tooth or tooth equivalent thereto was extracted from each healthy donor under local anesthesia, and periodontal ligament tissues were collected from a 1/3 region at the center of the root of the tooth. The obtained periodontal ligament tissues were enzymatically treated to isolate cells. The procedures described in Iwata et al., Regen. Ther., Vol. 9, pp. 38-44 (2018) were adopted as actual procedures.

The collected cells were cultured using $\alpha$-MEM (containing 10% FBS, L-glutamine, Gentacin, and fungizone) at 37°C in a 5% $CO_2$ environment for approximately 1 week and then subcultured. At the third passage, the cells were adjusted to $1 \times 10^5$ cells/tube using STEM-CELLBANKER(R) and cryopreserved in liquid nitrogen.

[Example 2] Measurement of alkaline phosphatase activity

ALP activity was measured for the cell population derived from each sample by the following method: the cell population was seeded at $1 \times 10^5$ cells/mL to a 96-well plate (100 $\mu$L: $1 \times 10^4$ cells/well). The cells were cultured in a basal medium ($\alpha$-MEM supplemented with 10% FBS) at 37°C in a 5% $CO_2$ environment for 2 days and then, after medium replacement, further cultured for 5 days. The medium was removed, and the cells were washed twice with 200 $\mu$L of physiological saline. 100 $\mu$L of a substrate buffer solution of LabAssay ALP (manufactured by FUJIFILM Wako Pure Chemical Corp.) was added thereto and incubated at 37°C for 5 minutes. Then, 80 $\mu$L of a reaction stop solution was added thereto, followed by the measurement of absorbance at 405 nm in a microplate reader. The ALP activity (U) of each cell population was calculated from each absorbance using a calibration curve prepared on the basis of the absorbance of standard solutions of LabAssay ALP.

ALP activity after calcification induction was measured for each cell population. The cell population was seeded at $1 \times 10^5$ cells/mL to a 96-well plate (100 $\mu$L: $1 \times 10^4$ cells/well). The cells were cultured in a basal medium at 37°C in a 5% $CO_2$ environment for 2 days and then, after medium replacement with a calcification induction medium ($\alpha$-MEM supplemented with 10% FBS, L-ascorbic acid phosphate magnesium salt n-hydrate, dexamethasone, and $\beta$-glycero-

phosphoric acid), further cultured at 37°C in a 5% $CO_2$ environment for 5 days. The medium was removed, and the cells were washed twice with 200 μL of physiological saline. 100 μL of a substrate buffer solution of LabAssay ALP (manufactured by FUJIFILM Wako Pure Chemical Corp.) was added thereto and incubated at 37°C for 5 minutes. Then, 80 μL of a reaction stop solution was added thereto, followed by the measurement of absorbance at 405 nm in a microplate reader. The ALP activity (U) of each cell population was calculated from each absorbance using a calibration curve prepared on the basis of the absorbance of standard solutions of LabAssay ALP.

Figurer 1 shows results of measuring the ALP activity of each cell population before calcification induction and after calcification induction. The cell populations derived from the tissues of P304 and P305 (hereinafter, these cell populations are simply referred to as "P304" and "P305", etc.), which had high ALP activity before calcification induction, exhibited markedly high ALP activity after the induction. On the other hand, ALP activity after the induction was not much increased in P307 and P309 having low ALP activity before the induction. This demonstrated that a cell population having high ALP activity before calcification induction causes marked increase in ALP activity after the induction.

[Example 3] Measurement of cellular proliferative capability

Each cell population before the induction was seeded at $1 \times 10^3$ cells to a 96-well plate and cultured in a basal medium at 37°C in a 5% $CO_2$ environment for 2 days and 4 days. Then, the medium was replaced with 100 μL of a fresh basal medium, to which 20 μL of Cell Titer 96 AQueous One Solution Cell Proliferation Assay (Promega Corp.) was then added and incubated at 37°C in a 5% $CO_2$ environment for 1 hour. Then, absorbance at 490 nm was measured using a microplate reader (Model 450; Bio-Rad Laboratories, Inc.). Detailed conditions of this measurement were set to the condition described in Iwata et al., J. Clin. Periodontol., Vol. 37, pp. 1088-1099 (2010).

Figurer 2 shows the cellular proliferative capability of each cell population. P304 and P305 exhibited sufficiently high cellular proliferative capability. This demonstrated that a cell population having high ALP activity before calcification induction has sufficiently high cellular proliferative capability.

[Example 4] Measurement of colony forming capability

100 cells of each cell population were seeded to a 60 cm$^2$ Petri dish and cultured in a basal medium at 37°C in a 5% $CO_2$ environment for 14 days. During this period, medium replacement was carried out twice a week. Then, the cells were stained with 0.5% crystal violet and washed twice with distilled water. After drying of the Petri dish, stained portions were counted. The number of cells that formed a colony to the seeded 100 cells, i.e., a colony count, was used as colony forming capability (%). Detailed conditions of this measurement were set to the conditions described in Iwata et al., J. Clin. Periodontol., Vol. 37, pp. 1088-1099 (2010).

Figurer 3 shows the colony forming capability of each cell population. P304 and P305 exhibited sufficiently high colony forming capability. This demonstrated that the cell population having ALP activity before calcification induction has colony forming capability.

[Example 5] Preparation of cell sheet

The cell population of P304 was used as a material of a cell sheet. The cell population was seeded at $3 \times 10^5$ to $4 \times 10^5$ cells to UpCell (manufactured by CellSeed Inc.) and cultured in a calcification induction medium at 37°C in a 5% $CO_2$ environment for 9 to 10 days. During this period, confirmation of the state of the cells and medium replacement were performed every 3 to 4 days. The medium was removed, and the cells were washed twice with 1 mL of physiological saline warmed to 37°C. Then, a cell sheet was recovered with a ring-shaped support. The recovered cell sheet was placed on another cell sheet already washed, and recovered as layers. Finally, a three-layer cell sheet product was prepared.

[Example 6] Preservation/transport stability test on cell sheet

The cell sheet prepared in Example 5 was studied for its stability under preservation/transport conditions. The cell sheet prepared in Example 5 was housed in a dedicated transport container containing α-MEM. The transport container with the cell sheet housed therein was left standing at ordinary temperature for 0 days and 7 days. The transport container thus left standing for 7 days was transported (by walking and train) for 1 hour under a condition of ordinary temperature. For a control, the cell sheet was similarly housed in a transport container containing PBS instead of α-MEM, which was then left standing for 7 days and transported for 1 hour under a condition of ordinary temperature.

A cell number, a cell survival rate, ALP activity, and an ALP-positive cell ratio of the cell sheet in each transport container were measured. The ALP activity was measured by the following approach: a portion of the cell sheet was recovered from the cell sheet product using a biopsy punch (φ8.0 mm) and placed in a 96-well plate (n = 3). Then, the ALP activity was measured under the same conditions as in Example 2 using LabAssay ALP.

The cell number and the cell survival rate were measured by the following approach: to the remaining portion of the cell sheet recovered using a biopsy punch, 200 µL of α-MEM was added, and further, 20 µL of collagenase was added, followed by incubation at 37°C in a 5% $CO_2$ environment. After 10 minutes, the cell sheet was taken out thereof, well dissociated by pipetting, and further incubated for 5 minutes. Then, 200 µL of 0.25% trypsin-EDTA was added thereto, and the cell sheet was dissociated by pipetting again and incubated for 5 minutes. The cell sheet was confirmed to become a cell suspension by sufficient dissociation, to which 600 µL of a cell washing solution (PBS + 10% FBS) was then added. The cells were transferred to a 1.5 mL tube and centrifuged at 500 g × 5 minutes at room temperature. After removal of a supernatant, a cell suspension was prepared by the addition of 1 mL of a cell washing solution again, and 15 µL thereof was transferred to a fresh 1.5 mL tube. 15 µL of trypan blue was added thereto, and the cells were dispersed by pipetting to confirm a cell number and survival rate.

The ALP-positive cell ratio was measured by the following approach: cells recovered from the cell sheet in the same manner as above were suspended in a cell washing solution. The cell suspension was divided into an ALP test solution and a control test solution. The ALP test solution was reacted by the addition of a phycoerythrin-labeled anti-ALP antibody, while the control test solution was reacted by the addition of phycoerythrin-labeled mouse nonspecific γG1 immunoglobulin. After incubation at 4°C for 30 minutes, the cells of each test solution were washed with a cell washing solution and fixed in 1% paraformaldehyde to prepare a cell sample. Each cell sample was assayed using flow cytometry, and its ALP-positive cell ratio (%) was calculated.

The conditions described in Washio, et al., Int. J. Oral Maxillofac. Implants, vol. 29, No. 1, e117-121 (2014) were used as the measurement conditions for the ALP activity, the cell number, the cell survival rate, and the ALP-positive cell ratio.

Table 1 shows the cell number, the cell survival rate, the ALP activity, and the ALP-positive cell ratio of the cell sheet in each transport container. The cell sheet preserved in α-MEM was shown to sufficiently maintain performance as a product even if preserved at ordinary temperature for 7 days and transported for 1 hour. On the other hand, the cell sheet immersed in PBS instead of α-MEM resulted in a cell survival rate of 0% by preservation at ordinary temperature for 7 days + transport for 1 hour.

[Table 1]

| | Value of standard | 0-day preservation | 7-day preservation | 7-day preservation + 1-hour transport |
|---|---|---|---|---|
| Cell number (cell/ product) | $1 \times 10^6$ | $1.3 \times 10^6$ | $1.8 \times 10^6$ | $1.2 \times 10^6$ |
| Cell survival rate (%) | $\geq 80$ | 99.2 | 99.4 | 97.4 |
| ALP activity (U) | $\geq 0.1$ | 3.36 | 3.03 | 4.12 |
| ALP-positive cell ratio (%) | $\geq 50$ | 86.2 | 82.6 | 75.1 |

All publications, patents, and patent applications cited herein are incorporated herein by reference in their entirety.

**Claims**

1. A cell population comprising mesenchymal stem cells derived from a dental or periodontal tissue, wherein alkaline phosphatase activity before calcification induction is 1 U or more.

2. The cell population according to claim 1, wherein alkaline phosphatase activity after calcification induction is 5 U or more.

3. The cell population according to claim 1, wherein the mesenchymal stem cells are mesenchymal stem cells derived from periodontal ligament.

4. A cell sheet comprising a cell population comprising mesenchymal stem cells derived from a dental or periodontal tissue, wherein alkaline phosphatase activity after calcification induction of the cell population is 5 U or more.

5. The cell sheet according to claim 4, wherein alkaline phosphatase activity before calcification induction of the cell population is 1 U or more.

6. A method for producing a cell sheet, comprising the following steps (a) to (c):

(a) obtaining a cell population comprising mesenchymal stem cells derived from a dental or periodontal tissue and having alkaline phosphatase activity;
(b) culturing the cell population in a calcification induction medium, and proliferating the cell population on a plastic substrate; and
(c) recovering and washing a sheet-shaped cell population formed on the plastic substrate.

7. The method according to claim 6, wherein alkaline phosphatase activity of the cell population in the step (a) is 1 U or more.

8. The method according to claim 6 wherein alkaline phosphatase activity after calcification induction of the cell population is 5 U or more.

9. A method for preserving and/or transporting a cell sheet comprising a cell population comprising mesenchymal stem cells derived from a dental or periodontal tissue, wherein the cell population has high alkaline phosphatase activity, and the cell sheet is maintained in alpha modified Eagle's minimum essential medium at ordinary temperature.

10. The method according to claim 9, wherein the cell sheet is capable of being maintained in a usable state for 7 days or longer in the alpha modified Eagle's minimum essential medium at ordinary temperature.

11. The method according to claim 9 or 10, wherein the cell sheet is a cell sheet according to claim 4 or 5 or a cell sheet produced by a method according to any one of claims 6 to 8.

# Fig. 1

# Fig. 2

# Fig. 3

## INTERNATIONAL SEARCH REPORT

| International application No. |
|---|
| **PCT/JP2022/042503** |

**A. CLASSIFICATION OF SUBJECT MATTER**

***C12N 5/0775*** (2010.01)i
FI: C12N5/0775

According to International Patent Classification (IPC) or to both national classification and IPC

**B. FIELDS SEARCHED**

Minimum documentation searched (classification system followed by classification symbols)

C12N5/0775

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2010-252778 A (SHIZUI BANK KK) 11 November 2010 (2010-11-11) | 1-3 |
|   | claim 1, paragraphs [0026], [0029], [0030], [0033] |   |
| Y | claim 1, paragraphs [0026], [0029], [0030], [0033] | 1-11 |
| Y | WO 2017/073656 A1 (KANEKA CORPORATION) 04 May 2017 (2017-05-04) | 1-11 |
|   | claim 23, paragraphs [0124], [0173] |   |
| Y | JP 2011-520434 A (BONE THERAPEUTICS S.A.) 21 July 2011 (2011-07-21) | 1-11 |
|   | examples 1-6, table 7 |   |
| A | MORITANI, Y. et al. Spheroid culture enhances osteogenic potential of periodontal ligament mesenchymal stem cells. Journal of Periodontal Research. 2018, vol. 53, pp. 870-882 | 1-11 |
|   | entire text |   |

☐ Further documents are listed in the continuation of Box C.    ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **27 December 2022** | **17 January 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2022/042503**

| Patent document cited in search report | | | Publication date (day/month/year) | Patent family member(s) | Publication date (day/month/year) |
|---|---|---|---|---|---|
| JP | 2010-252778 | A | 11 November 2010 | (Family: none) | |
| WO | 2017/073656 | A1 | 04 May 2017 | US 2018/0362922 A1 claim 23, paragraphs [0192], [0248] | |
| JP | 2011-520434 | A | 21 July 2011 | US 2011/0262404 A1 examples 1-6, table 7 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005103233 A
- JP 2021188216 A
- JP 2211865 A
- JP 2018121568 A

**Non-patent literature cited in the description**

- **M. DOMINICI, MD et al.** *Cytotherapy,* 2006, vol. 8, 315-317
- **IWATA et al.** *J. Clin. Periodontol.,* 2010, vol. 37, 1088-1099
- **WASHIO et al.** *Int. J. Oral Maxillofac. Implants,* 2014, vol. 29 (1), e117-121
- **IWATA et al.** *Regen. Ther.,* 2018, vol. 9, 38-44
- **IWATA et al.** *J. Clin. Periodontol,* 2010, vol. 37, 1088-1099